# FASCICULE DE BREVET EUROPEEN

(11) **EP 2 225 416 B1**
(45) Date de publication et mention de la délivrance du brevet: **13.07.2011**
(21) Numéro de dépôt: 08871946.3
(22) Date de dépôt: 29.10.2008
(51) Int. Cl.: D21C 9/18, B30B 9/00

(54) **PROCÉDÉ DE CONVERSION DE SOLUTIONS LIGNOCELLULOSIQUES A HAUTE TENEUR EN MATIÈRE SÈCHE**
VERFAHREN ZUR UMWANDLUNG VON LIGNOZELLULOSE-LÖSUNGEN MIT HOHEM TROCKENMATERIALANTEIL
METHOD FOR CONVERTING LIGNOCELLULOSIC SOLUTIONS HAVING A HIGH DRY-MATERIAL CONTENT

(30) Priorité: 20.12.2007 FR 0709115
(43) Date de publication de la demande: 08.09.2010
(73) Titulaire: IFP Energies nouvelles, 92852 Rueil-Malmaison Cedex (FR)
(72) Inventeur: ROLLAND, Matthieu, F-69390 Vernaison (FR)
(86) Numéro de dépôt international: PCT/FR2008/001525
(87) Numéro de publication internationale: WO 2009/095549

(56) Documents cités:
- EP-A- 0 358 837
- EP-A- 0 858 880
- EP-A- 1 690 944
- WO-A-90/00213
- WO-A-2005/025846
- FR-A- 2 347 489

## Description

### Domaine technique

L'invention concerne un procédé de conversion de solution aqueuses de solides lignocellulosiques comprenant une teneur en solides comprise entre 1 et 20% de matière sèche, ledit procédé comprenant une étape a) de compression de ladite solution de manière à séparer la phase liquide présente dans et entre les solides de la phase solide compressée et une étape b) d'extraction d'au moins la phase liquide, ladite phase liquide étant ensuite traitée / homogénéisée par des traitements thermiques et / ou chimiques et réinjectée sur la phase solide compressée.

L'invention concerne donc le domaine des procédés de conversion de solution de solides lignocellulosiques à teneur élevée en solides, exprimées en pourcentage de matière sèche, lesdits procédés étant particulièrement intéressants dans le cas de réactions limitées par les réactifs et / ou inhibées par les produits de réaction.

On entend par matière sèche, la quantité de matière insoluble initialement ajoutée par rapport au poids total du mélange réactionnel.

Selon la présente invention, la matière sèche est constituée de matière insoluble et en particulier de solides granulaires qui en solution aqueuse se présentent sous la forme de grumeaux compressibles. Ces solides granulaires sont avantageusement constitués de :
- biomasse lignocellulosique avant ou après pré-traitement et en particulier la pâte à papier, la paille et/ou le son de céréales, la bagasse de cannes à sucre, la pulpe de betterave ou les copeaux de bois.

Les solutions aqueuses de matériaux lignocellulosiques ont une rhéologie qui dépend de la teneur en solide, et plus particulièrement de la teneur en cellulose qui gonfle lorsqu'elle est mise en solution.

La pâte à papier, qui contient 70% de cellulose, est travaillée dans les usines de pâte à papier à des concentrations en solides inférieures à 4 - 5% MS (matière sèche). A ces concentrations, les solutions se comportent comme des liquides et sont mises en oeuvres avec les technologies classiques et simples : pompes, tuyaux, réacteurs de type cuve, agitateurs. A des concentrations plus fortes, les mélanges eaux - pâte à papier se compose de grumeaux de solide imbibés d'eau en contact les uns avec les autres et collant peu les uns aux autres. Le mélange n'a ni les propriétés d'écoulement des liquides, ni celles de solides secs divisés. En particulier l'agitation est très difficile : soit les internes de mélange mettent la pâte en mouvement de type bloc (pas d'agitation), soit ils cisaillent la pâte en plusieurs blocs. Les mises en oeuvre de ces mélanges sont délicates : les pompes sont remplacées par exemple par des vis sans fin ou des tapis ou des dispositifs à godets. Ce type de mélange est en fait mis en oeuvre de manière transitoire, soit lors d'un séchage et l'obtention d'un solide sec (60-90%MS), soit lors d'une mise en solution d'un solide sec.

Les autres biomasses lignocellulosiques contiennent généralement 30% de cellulose, de sorte qu'elles gonflent moins et peuvent être opérées comme des liquides jusqu'à des teneurs en matière sèche de 10% environ.

L'objet de la présente invention est de fournir un procédé de conversion de solution de solides lignocellulosiques comprenant une teneur élevée en solides exprimée en pourcentage de matière sèche de manière à permettre des traitements dans des gammes où les réactions sont habituellement incomplètes, où la répartition des réactifs et où les phénomènes d'inhibition par concentration des produits conduisent à des cinétiques plus lentes et/ou des rendements de réactions moins intéressants.

L'objet de l'invention est la mise en oeuvre dudit procédé pour la réaction d'hydrolyse enzymatique.

### Art antérieur :

Le brevet US 7 267 049 B2 décrit un appareil de type presse pour réduire la teneur en eau d'une pâte au moyen d'un système à vis. La phase liquide et la phase concentrée en solide sont extraites séparément.
La présente invention utilise cette propriété des systèmes à vis pour favoriser les réactions en milieu pâteux mais se différencie notamment en ce que la séparation est temporaire et le solide est à nouveau mélangé au liquide homogénéisé de manière à améliorer le rendement et/ou la sélectivité de la réaction.

Le brevet US 7 217 340 B2 appartient à un autre domaine que celui de la conversion de solution de solides lignocellulosiques comprenant une teneur élevée en solides et décrit une composition adoucissante et son utilisation dans le traitement des matériaux cellulosiques contenant des fibres tels que le papier, les textiles et les échantillons de tissus. En particulier, dans ledit brevet, la teneur en fibre de la pâte à papier est augmentée jusqu'à 7% de matière sèche par un pressage sous vide et jusqu'à 25% de matière sèche par passage dans des rouleaux comprimant la pâte en une feuille. Il s'agit donc d'un traitement final.

L'hydrolyse enzymatique est une réaction enzymatique de conversion du substrat (solide) cellulosique en cellobiose (produit intermédiaire) puis en glucose (sucre final). Les enzymes sont les plus performantes dans une gamme étroite de température et de pH, généralement 50-60°C et pH = 4.5-5.5. Les réactions sont inhibés par le sucre final et très fortement inhibé par le cellobiose, les inhibitions augmentant avec les concentrations locales.

Concernant l'hydrolyse enzymatique de biomasse lignocellulosique, la teneur maximale en solide exprimée en pourcentage de matière sèche généralement admise est de 2 à 5%. Au delà, les problématiques suivantes surviennent :
- augmentation locale des teneurs en cellobiose, intermédiaire de réaction qui inhibe les réactions d'hydrolyse de la cellulose
- augmentation locale des teneurs en glucose, produit de la réaction, qui inhibe aussi les réactions de conversion
- inhomogénéité de répartition des enzymes
- inhomogénéité du pH conduisant à des opérations plus ou moins performantes selon les endroits
- inhomogénéité des températures

Toutes ces limitations augmentent avec la teneur en matière sèche. Or il est économiquement intéressant de pouvoir travailler à plus forte teneur en matière initiale (plus de cellulose), car cela conduit à des concentrations en sucres plus élevées et en conséquence en des teneurs en alcool plus élevées. Cela permet de réduire les coûts de distillation vers de l'alcool pur, significatifs dans le prix de revient.

### Objet et intérêt de l'invention

La présente invention concerne un procédé de conversion de solution aqueuse de solides lignocellulosiques comprenant une teneur en solides comprise entre 1 et 20% de matière sèche, ledit procédé comprenant une étape a) de compression de ladite solution de manière à séparer la phase liquide présente dans et entre les solides de la phase solide compressée et une étape b) d'extraction d'au moins la phase liquide, ladite phase liquide étant ensuite traitée / homogénéisée par des traitements thermiques et / ou chimiques et réinjectée sur la phase solide compressée.

Le procédé selon la présente invention permet donc d'homogénéiser les conditions de réactions sur des pâtes formées de grumeaux compressibles par un renouvellement complet de la phase liquide présente dans les grumeaux et son remplacement par un liquide homogénéisé (concentrations, pH, température, ...).

Les grumeaux sont pressés de manière à évacuer le liquide présent dans les pores des grumeaux. La phase liquide récupérée lors du pressage est alors mélangée facilement avec une homogénéisation de tous les paramètres influant sur la réaction : concentrations, pH, température, ... La phase liquide homogénéisée est remise en contact avec les grumeaux compressés constituant la phase solide compressée qui réabsorbent en quelques secondes tout liquide avec lequel ils sont mis en contact. Tous les grumeaux sont alors exposés à des conditions réactionnelles identiques et plus favorables à la réaction qu'avant la mise en oeuvre du procédé.

Ledit procédé permet également d'obtenir des conditions réactionnelles identiques quelque soit la taille des grumeaux.

### Description des figures:

La figure 1 représente le mode de réalisation du réacteur à piston dans lequel la réaction de conversion de solution aqueuse lignocellulosique et l'étape a) de compression du procédé selon l'invention ont lieu.
La figure 2 représente le mode de réalisation du réacteur à piston dans lequel le piston est un piston rotatif et illustre également les moyens de malaxage consistant en des protubérances faisant saillie sur une partie au moins de la hauteur de la phase solide compressée.
La figure 3 représente le mode de réalisation du réacteur à vis dans lequel la réaction de conversion de solution aqueuse lignocellulosique et l'étape a) de compression du procédé selon l'invention ont lieu.
La figure 4 représente les différents modes de réinjection et de dispersion de la phase solide issue de l'étape b) d'extraction en tête du réacteur à vis, la vis en fond de cuve n'étant pas représentée sur la figure.

### Description détaillée de l'invention :

L'invention décrit un procédé de conversion de solution aqueuse de solides lignocellulosiques selon la revendication 1 comprenant une teneur en solides comprise entre 1 et 20%, de préférence comprise entre 1 et 15% et de manière très préférée supérieure à 8% et de manière encore plus préférée compris entre 8 et 15% de matière sèche, ledit procédé comprenant une étape a) de compression de ladite solution de manière à séparer la phase liquide présente dans et entre les solides de la phase solide compressée et une étape b) d'extraction d'au moins la phase liquide, ladite phase liquide étant ensuite homogénéisée par des traitements thermiques et/ou chimiques et re-mélangée à la phase solide.

L'étape a) de compression est avantageusement mise en oeuvre selon un axe de compression vertical ou dans une vis.

Ladite étape a) est également avantageusement réalisée sous agitation pour améliorer l'homogénéisation.

La phase liquide collectée est avantageusement injectée de manière dispersée, de manière à asperger uniformément la surface supérieure de la phase solide. La dispersion est avantageusement réalisée par un réseau de buses d'alimentation qui génère des gouttes uniformément réparties.

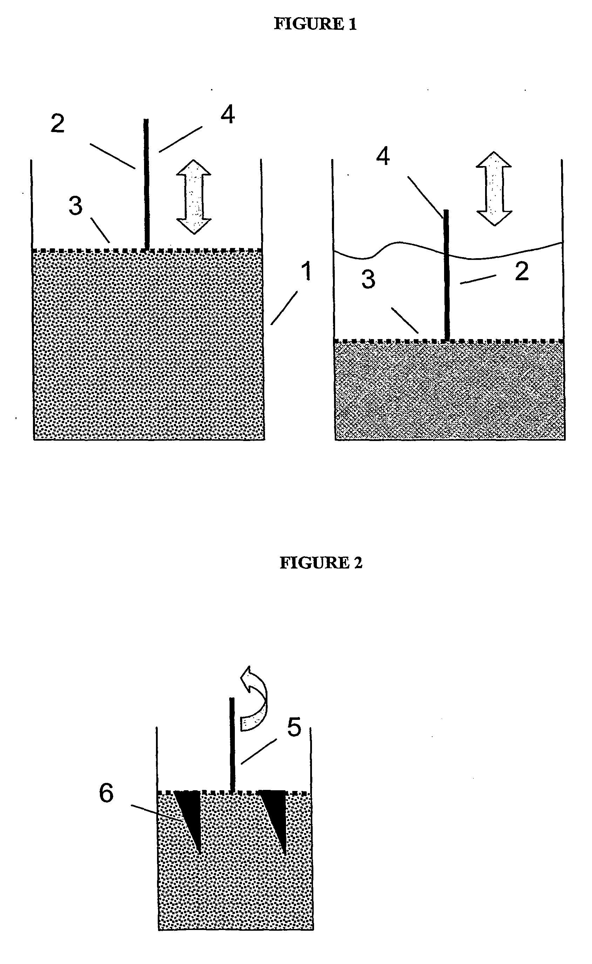

La description de l'invention est faite en se rapportant aux figures 1 à 4.

Selon un premier mode de réalisation représenté sur la figure 1, l'étape a) de compression du procédé selon l'invention est avantageusement mise en oeuvre dans un réacteur (1) comportant un piston de compression perforé (2), ledit piston de compression comportant 3 éléments distincts :
- une grille perforée (3) laissant passer le liquide et bloquant les grumeaux, la taille moyenne des perforations étant avantageusement comprise entre 1 à 3 fois la taille des grumeaux.
- un support de grille et
- un axe de compression (4) perpendiculaire à ladite grille.

De préférence, Il n'est pas nécessaire que la grille (3) arrête la totalité des grumeaux. Le pas le plus fin de la grille est de 500 µm. Pour un pas de grille inférieur, le flux de liquide sera faible et la descente du piston trop lente. La grille (3) est avantageusement réalisée avec une plaque perforée ou avec un grillage métallique tissé ou avec un matériau poreux (fritté métallique, céramique) ou une membrane polymère (hydrophile ou hydrophobe) Le support de grille a pour fonction de transmettre les efforts mécaniques entre la grille et l'axe de compression perpendiculaire à ladite grille, de maintenir la grille horizontale, d'assurer l'étanchéité au entre les parois et le piston, et de laisser passer le liquide.

Selon ce premier mode de réalisation, le piston (2) est régulièrement descendu et remonté dans le sens de la flèche indiqué sur la figure 1. Après la descente du piston, la phase liquide se retrouve au dessus du piston, la phase solide compressée en dessous.

La solution aqueuse de solides lignocellulosiques peut avantageusement être agitée : l'agitation peut être mise en oeuvre de deux manières différentes : soit le piston (2) de compression peut comporter un agitateur (non représenté sur la figure 1) dont l'axe d'entraînement est interne et coaxial à l'axe de compression dudit piston (2), soit, le piston de compression est un piston de compression rotatif (5) équipé de protubérances (6) faisant saillie sur une partie au moins de la hauteur de la solution aqueuse de solides lignocellulosiques.

La phase liquide est naturellement au moins partiellement homogénéisée lors de l'étape a) de compression du fait de son passage a travers les grumeaux de solides lignocellulosiques et à travers la grille (3).
Avantageusement, l'étape b) d'extraction de la phase liquide est effectuée par un prélèvement de la phase liquide à l'extérieur du réacteur (1) puis réinjection au moyen d'une pompe et d'un injecteur. Le débit de prélèvement et d'injection est ajusté de manière à ce que le volume du réacteur soit pompé au moins 3 fois, et de préférence 10 fois avant relevage du piston.

De manière à améliorer l'homogénéité de la phase liquide, l'axe de compression (4) dudit piston de compression rotatif peut avantageusement être équipé de pâles au dessus de la grille.

En effet, l'effet de pompage est maximal au début, lors de la première heure. Ensuite, avec avancement de la réaction, la teneur en solide diminue fortement et l'effet de pompage est moins intéressant. Il peut donc être avantageux de relever totalement le piston et d'opérer de manière habituelle à savoir en utilisant uniquement un agitateur classique.

Dans le cas ou le procédé selon l'invention est mis en oeuvre dans le réacteur a piston décrit si dessus, la durée entre les pompages est avantageusement comprise, pour la première heure entre 1 et 10 minutes et de préférence entre 2 et 7 minutes et ensuite entre 5 et 120 minutes, et de préférence entre 10 et 60 minutes.

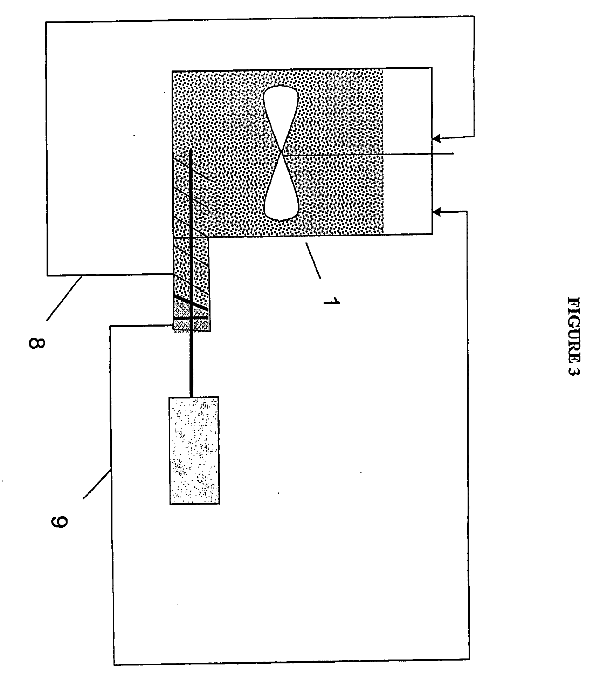

Selon un deuxième mode de réalisation illustré sur la figure 3, l'étape a) de compression est mise en oeuvre dans un réacteur (1) comprenant une vis (7) en fond de cuve dans laquelle ladite étape a) de compression a lieu.
Dans ce cas, la collecte séparée de la phase liquide (conduite 8) et de la phase solide compressée (conduite 9) est avantageusement réalisée à l'issue de l'étape a) de compression.
En effet, la phase solide compressée est également avantageusement extraite de préférence en continu lors de l'étape b) d'extraction et réinjectée en tête dudit réacteur par la conduite (9).

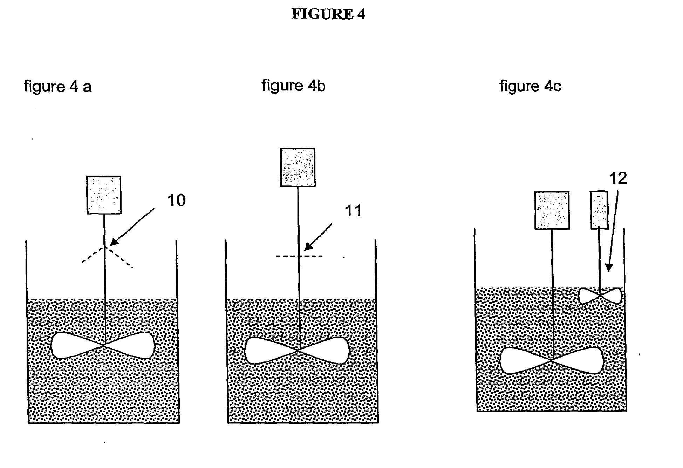

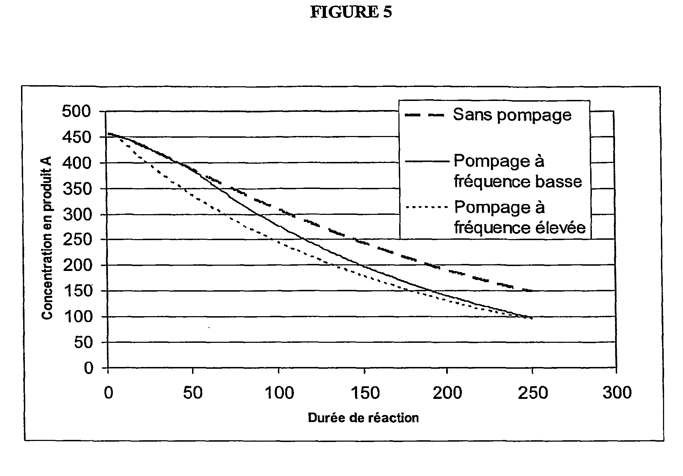

Selon un premier mode de réalisation préféré dudit deuxième mode de réalisation représenté sur la figure 4a, ladite phase solide compressée est avantageusement réinjectée et dispersée en tête de réacteur, de préférence au moyen d'un cône de dispersion (10) statique ou rotatif, de demi angle au sommet compris entre 30 et 60° et de diamètre compris entre 1/3 et ¼ du diamètre du réacteur ou de manière préférée, au moyen d'un plateau de dispersion rotatif (11), de diamètre compris entre 1/3 et ¼ du diamètre du réacteur, ledit plateau étant entraîné par l'axe d'entraînement de l'agitateur, représenté sur la figure 4b.

Selon un autre mode de réalisation préféré dudit deuxième mode de réalisation représenté sur la figure 4c, ladite phase solide compressée est avantageusement réinjectée et dispersée en tête de réacteur au moyen d'un mélangeur secondaire (12) dédié à la mise en contact intime de la phase solide avec la phase liquide.

La phase liquide collectée est avantageusement injectée de manière dispersée, de manière à asperger uniformément la surface supérieure de la phase solide. La dispersion est avantageusement réalisée par un réseau de buses d'alimentation qui génère des gouttes uniformément réparties.

Dans le cas ou le procédé selon l'invention est mis en oeuvre dans le réacteur a vis décrit ci dessus, le taux de renouvellement par la vis est, pendant les deux première heures, compris entre un volume de réacteur toutes les minutes et un volume de réacteur toutes les 30 minutes et de préférence compris entre un volume de réacteur toutes les 5 minutes et un volume de réacteur toutes les 20 minutes et ensuite compris entre un volume de réacteur toutes les 20 minutes et un volume de réacteur toutes les 60 minutes et de préférence compris un volume de réacteur toutes les 30 minutes et un volume de réacteur toutes les 50 minutes.

Quelque soit le mode de réalisation de l'étape de compression, et conformément à l'invention, la phase liquide est homogénéisée par des traitements thermiques et / ou chimiques avant d'être re-mélangée à la phase solide.
Les traitements thermiques et/ou chimiques avantageusement réalisés sur la phase liquide extraite sont les suivants :
- une régulation de température par des moyens connus de l'homme du métier,
- une régulation du pH, par des moyens connus de l'homme du métier,
- un ajout de réactifs tel que par exemple des antibiotiques.

L'objet de l'invention est la mise en oeuvre dudit procédé pour la réaction d'hydrolyse enzymatique.
Dans ce cas, la réaction d'hydrolyse enzymatique décrite ci dessus a lieu dans le réacteur à piston ou à vis décrit ci dessus.

Avantageusement, une réaction de fermentation alcoolique a également lieu dans ledit réacteur à piston ou a vis simultanément à la réaction d'hydrolyse enzymatique.

La biomasse lignocellulosique peut avantageusement subir une étape de prétraitement telle que par exemple des opérations chimiques, thermiques et mécaniques destinées à favoriser les rendements de la réaction d'hydrolyse enzymatique. Ces prétraitements nécessitent la manipulation de pâtes plus ou moins sèches selon les cas. De tels prétraitements peuvent être par exemple, les cuissons en phase acide ou basique, les opérations de compression - détente (explosion à la vapeur, procédé AFEX), ... Généralement, les produits issus des étapes de prétraitements sont des solides de type grains ou grumeaux, ou des pâtes à forte teneur en eau. L'invention ouvre ainsi des potentialités sur des traitements intermédiaires avec production ou mise en oeuvre de pâte à forte teneur en matière sèche.

Avantageusement, les traitements thermiques et/ou chimiques réalisés sur la phase liquide extraite à l'issu de la réaction d'hydrolyse enzymatique sont les suivants :
- la phase liquide peut avantageusement passer dans un procédé de conversion de la cellobiose en glucose de manière a réaliser une hydrolyse enzymatique additionnelle à l'extérieur du réacteur.
- la phase liquide peut avantageusement après changement des conditions opératoires selon des techniques connues de l'homme du métier passer dans un procédé séparé de fermentation, l'effluent issu de la fermentation étant ensuite réinjecté en tête de réacteur après réajustements des conditions opératoires (pH, températures...) aux conditions opératoires de l'hydrolyse enzymatiques selon des techniques connues de l'homme du métier.

### Exemples

### Exemple 1 : Hydrolyse enzymatique

La réaction d'hydrolyse enzymatique est mise en oeuvre dans le réacteur à vis d'extraction en fond en fond de cuve selon le procédé selon l'invention, la solution de solides lignocellulosique étant constituée d'une solution de pâte a papier contenant 15% de matière insoluble, l'ajout de la matière première au fur et à mesure

Les conditions opératoires dans le réacteur à vis sont les suivantes :
- température du réacteur 55°C,
- pH = 4,8

Taux de renouvellement par la vis est de :
- pendant les deux premières : 1 volume de réacteur en 20 minutes
- et ensuite : 1 volume de réacteur en 50 minutes.

La phase liquide extraite subit un traitement d'ajustement du pH selon une technique connue de l'homme du métier.

### Exemple 2 : Hydrolyse enzymatique

La réaction d'hydrolyse enzymatique est mise en oeuvre dans le réacteur a piston selon le procédé selon l'invention, la solution de solides lignocellulosique étant constituée d'une solution de copeaux de bois contenant 13% de matière insoluble, l'ajout de la matière première au fur et à mesure

Les conditions opératoires dans le réacteur à vis sont les suivantes :
- température du réacteur 55°C,
- pH = 4,8
   la durée entre les pompages étant la suivante :
- la première heure : 1 pompage toutes les 5 minutes
- ensuite : 1 pompage toutes les 30 minutes

### Exemple 3 : Modèle avec limitation diffusionnelle en réactif

Cette simulation met en évidence l'efficacité du procédé selon l'invention qui permet d'uniformiser la concentration en réactif A quand le procédé de conversion est mis en oeuvre dans un réacteur à piston par rapport à un procédé ne comportant pas d'étape b) d'extraction de la phase liquide, ni d'homogénéisée par des traitements thermiques et / ou chimiques et sa re- injection sur la phase solide.

La réaction chimique mise en oeuvre dans le réacteur a piston décrit ci dessus est une réaction chimique dont la cinétique est d'ordre 1. On mesure la concentration d'un réactif A en solution dans la phase liquide. La diffusion du réactif A dans le grumeau est lente et limite le taux de disparition du réactif A : le coeur du grumeaux ne travaille pas de manière optimale.

La modélisation montre qu'un pompage régulier permet d'améliorer les rendements (c'est à dire que une plus grande quantité de A est consommée dans le même temps ou autant de A est consommé en moins de temps), et ce d'autant plus que la fréquence de pompage est plus élevée.

Le pressage des grumeaux selon le procédé selon l'invention permet donc d'uniformiser la concentration en A dans le grumeau et à l'extérieur du grumeau, de sorte que les limitations diffusionnelles qui limitent la réaction sont minimisées.

La figure 4 représente la concentration en produit A en fonction de la durée de réaction selon l'exemple 3.

## Revendications

1. Procédé de conversion de solutions aqueuses de solides lignocellulosiques comprenant une teneur en solides comprise entre 1 et 20% de matière sèche, ledit procédé comprenant une étape a) de compression de ladite solution de manière à séparer la phase liquide présente dans et entre les solides, de la phase solide compressée et une étape b) d'extraction d'au moins la phase liquide, ladite phase liquide étant ensuite homogénéisée par des traitements thermiques et / ou chimiques et re-mélangée à la phase solide, ledit procédé mettant en oeuvre la réaction d'hydrolyse enzymatique.

2. Procédé selon la revendication 1 dans lequel l'étape a) de compression est mise en oeuvre selon un axe de compression vertical.

3. Procédé selon la revendication 2 dans lequel l'étape a) de compression est mise en oeuvre dans un réacteur comportant un piston de compression perforé, ledit piston de compression comportant 3 éléments distincts :
- une grille perforée
- un support de grille et
- un axe de compression perpendiculaire à ladite grille.

4. Procédé selon l'une des revendications 1 à 3 dans lequel l'étape a) de compression est réalisée sous agitation.

5. Procédé selon la revendication 4 dans lequel l'agitation est réalisée au moyen d'un agitateur dont l'axe d'entraînement est interne et co-axial à l'axe de compression dudit piston.

6. Procédé selon la revendication 4 dans lequel ledit piston de compression est un piston de compression rotatif équipé de protubérances faisant saillie sur une partie au moins de la hauteur de la solution aqueuse de solides lignocellulosiques.

7. Procédé selon la revendication 6 dans lequel l'axe dudit piston de compression rotatif est équipé de pales au dessus de la grille perforée, dans la phase liquide.

8. Procédé selon la revendication 1 dans lequel l'étape a) de compression est mise en oeuvre dans une vis.

9. Procédé selon la revendication 8 dans lequel l'étape a) de compression est mise en oeuvre dans un réacteur comprenant une vis.

10. Procédé selon la revendication 9 dans lequel la phase solide compressée est également extraite lors de l'étape b) d'extraction et réinjectée en tête dudit réacteur.

11. Procédé selon la revendication 10 dans lequel la phase solide compressée est réinjectée et dispersée en tête de réacteur.

12. Procédé selon la revendication 11 dans lequel la phase solide compressée est réinjectée au moyen d'un cône de dispersion statique ou rotatif, de demi angle au sommet compris entre 30 et 60° et de diamètre compris entre 1/3 et ¼ du diamètre du réacteur.

13. Procédé selon la revendication 11 dans lequel la phase solide compressée est réinjectée au moyen d'un plateau de dispersion rotatif, de diamètre compris entre 1/3 et ¼ du diamètre du réacteur, ledit plateau étant entraîné par l'axe d'entraînement de l'agitateur.

14. Procédé selon l'une des revendications 1 à 13 dans lequel une réaction de fermentation alcoolique a également lieu simultanément à la réaction d'hydrolyse enzymatique.

15. Procédé selon la revendication 14 dans lequel la phase liquide passe dans un procédé de conversion de la cellobiose en glucose de manière à réaliser une hydrolyse enzymatique additionnelle à l'extérieur dudit réacteur.

16. Procédé selon la revendication 14 dans lequel la phase liquide passe dans un procédé séparé de fermentation, l'effluent issu de la fermentation étant ensuite réinjecté en tête de réacteur.

## Claims

1. A process for the conversion of aqueous solutions of lignocellulosic solids comprising a solids content of between 1 and 20% of dry material, said process comprising a step a) for compression of said solution so as to separate the liquid phase present in and between the solids from the compressed solid phase and a step b) for extraction of at least the liquid phase, said liquid phase then being homogenised by heat and/or chemical treatments and re-mixed with the solid phase, said process carrying out the enzymatic hydrolysis reaction.

2. A process according to claim 1 wherein the compression step a) is carried out in accordance with a vertical compression axis.

3. A process according to claim 1 wherein the compression step a) is carried out in a reactor comprising a perforated compression piston, said compression piston comprising three distinct components:
- a perforated grill,
- a grill support, and
- a compression shaft which is perpendicular to said grill.

4. A process according to one of claims 1 to 3 wherein the compression step a) is performed with agitation.

5. A process according to claim 4 wherein agitation is effected by means of an agitator axis whose drive shaft is internal and coaxial with the compression shaft of the piston.

6. A process according to claim 4 wherein said compression piston is a rotary compression piston equipped with protuberances projecting over a part at least of the height of the aqueous solution of lignocellulosic solids.

7. A process according to claim 6 wherein the shaft of said rotary compression piston is equipped with blades above the perforated grill in the liquid phase.

8. A process according to claim 1 wherein the compression step a) is carried out in a screw.

9. A process according to claim 8 wherein the compression step a) is carried out in a reactor comprising a screw.

10. A process according to claim 9 wherein the compressed solid phase is also extracted in the extraction step b) and reinjected at the head of the reactor.

11. A process according to claim 10 wherein the compressed solid phase is reinjected and dispersed at the head of the reactor.

12. A process according to claim 11 wherein the compressed solid phase is reinjected by means of a static or rotary dispersion cone, with a half-angle at the top of between 30 and 60° and of a diameter between 1/3 and 1/4 of the diameter of the reactor.

13. A process according to claim 11 wherein the compressed solid phase is reinjected by means of a rotary dispersion plate of a diameter of between 1/3 and 1/4 of the diameter of the reactor, said plate being driven by the drive shaft of the agitator.

14. A process according to one of claims 1 and 13 wherein an alcoholic fermentation reaction also takes place simultaneously with the enzymatic hydrolysis reaction.

15. A process according to claim 14 wherein the liquid phase passes into a process for conversion of the cellobiose into glucose so as to effect additional enzymatic hydrolysis at the outside of said reactor.

16. A process according to claim 14 in which the liquid phase passes into a separate fermentation process, the effluent from the fermentation operation then being reinjected at the head of the reactor.

## Patentansprüche

1. Verfahren zur Umwandlung von wässrigen Lösungen lignocellulosehaltiger Feststoffe, die einen Feststoffgehalt im Bereich zwischen 1 und 20 % Trockensubstanz umfassen, wobei das Verfahren einen Schritt a) zur Verdichtung dieser Lösung, um die flüssige Phase, die in und zwischen den Feststoffen vorhanden ist, von der verdichteten festen Phase zu trennen und einen Schritt b) zur Extraktion mindestens der flüssigen Phase umfasst, wobei diese flüssige Phase anschließend durch Wärmebehandlungen und/oder chemische Behandlungen homogenisiert und der festen Phase wieder beigemischt wird, wobei bei dem Verfahren eine enzymatische Hydrolysereaktion durchgeführt wird.

2. Verfahren nach Anspruch 1, wobei Schritt a) zur Verdichtung gemäß einer vertikalen Verdichtungsachse durchgeführt wird.

3. Verfahren nach Anspruch 2, wobei Schritt a) zur Verdichtung in einem Reaktor durchgeführt wird, der einen perforierten Verdichtungskolben umfasst, wobei der Verdichtungskolben 3 verschiedene Elemente umfasst:
- ein perforiertes Gitter
- einen Gitterhalter und
- eine Verdichtungsachse senkrecht zu diesem Gitter.

4. Verfahren nach einem der Ansprüche 1 bis 3, wobei der Schritt a) zur Verdichtung unter Rühren ausgeführt wird.

5. Verfahren nach Anspruch 4, wobei das Rühren mittels eines Rührers ausgeführt wird, dessen Antriebsachse innerhalb der und koaxial zur Verdichtungsachse des Kolbens liegt.

6. Verfahren nach Anspruch 4, wobei der Verdichtungskolben ein Drehverdichtungskolben ist, der mit Vorsprüngen ausgestattet ist, die mindestens auf einem Teil der Höhe der wässrigen Lösung der lignocellulosehaltigen Feststoffe hervorragen.

7. Verfahren nach Anspruch 6, wobei die Achse des Drehverdichtungskolbens mit Schaufeln oberhalb des perforierten Gitters in der flüssigen Phase ausgestattet ist.

8. Verfahren nach Anspruch 1, wobei Schritt a) zur Verdichtung in einer Schraube durchgeführt wird.

9. Verfahren nach Anspruch 8, wobei Schritt a) zur Verdichtung in einem Reaktor durchgeführt wird, der eine Schraube umfasst.

10. Verfahren nach Anspruch 9, wobei die verdichtete feste Phase in Schritt b) zur Extraktion ebenfalls extrahiert wird und am Kopf des Reaktors reinjiziert wird.

11. Verfahren nach Anspruch 10, wobei die verdichtete feste Phase am Kopf des Reaktors reinjiziert und dispergiert wird.

12. Verfahren nach Anspruch 11, wobei die verdichtete feste Phase mittels eines statischen oder drehenden Dispersionskegels mit einem Halbwinkel an der Spitze im Bereich zwischen 30° und 60 und mit einem Durchmesser im Bereich zwischen 1/3 und 1/4 des Reaktordurchmessers reinjiziert wird.

13. Verfahren nach Anspruch 11, wobei die verdichtete feste Phase mittels einer drehenden Dispersionsplatte mit einem Durchmesser im Bereich zwischen 1/3 und 1/4 des Reaktordurchmessers reinjiziert wird, wobei die Platte von der Antriebsachse des Rührers angetrieben wird.

14. Verfahren nach Anspruch 1 bis 13, wobei gleichzeitig mit der enzymatischen Hydrolysereaktion außerdem eine alkoholische Fermentationsreaktion stattfindet.

15. Verfahren nach Anspruch 14, wobei die flüssige Phase ein Verfahren zur Umwandlung der Cellobiose in Glucose durchläuft, um eine zusätzliche enzymatische Hydrolyse außerhalb des Reaktors auszuführen.

16. Verfahren nach Anspruch 14, wobei die flüssige Phase ein getrenntes Fermentationsverfahren durchläuft, wobei der Abfluss, der aus der Fermentation entsteht, anschließend am Kopf des Reaktors reinjiziert wird.
